# EUROPÄISCHE PATENTSCHRIFT

(11) **EP 1 841 395 B1**
(45) Veröffentlichungstag und Bekanntmachung des Hinweises auf die Patenterteilung: **15.06.2011**
(21) Anmeldenummer: 06704276.2
(22) Anmeldetag: 19.01.2006
(51) Int. Cl.: A61H 33/06, F24F 6/06

(54) **VORRICHTUNG FÜR AUFGUSS- UND/ODER BEFEUCHTUNGSVORGÄNGE**
DEVICE FOR WETTING AND/OR HUMIDIFICATION PROCESSES
DISPOSITIF DESTINE A DES PROCESSUS D'INFUSION ET/OU D'HUMIDIFICATION

(30) Priorität: 27.01.2005 DE 202005001375 U
(43) Veröffentlichungstag der Anmeldung: 10.10.2007
(73) Patentinhaber: EOS-Werke Günther GmbH, 35759 Driedorf (DE)
(72) Erfinder: BASTIAN, Karl, Heinz, 35759 Driedorf (DE); GÜNTHER, Wolfgang, 35781 Weilburg (DE)
(74) Vertreter: Tappe, Hartmut
(86) Internationale Anmeldenummer: PCT/EP2006/000469
(87) Internationale Veröffentlichungsnummer: WO 2006/079479

(56) Entgegenhaltungen:
- DE-A1- 19 818 565
- DE-C1- 19 609 110
- DE-U1- 20 004 143
- DE-U1- 20 210 006
- US-A- 3 758 086
- PATENT ABSTRACTS OF JAPAN Bd. 2003, Nr. 12, 5. Dezember 2003 (2003-12-05) & JP 2005 282980 A (MATSUSHITA ELECTRIC IND CO LTD), 13. Oktober 2005 (2005-10-13)

## Beschreibung

Die Erfindung betrifft eine Vorrichtung ,geeignet Aufguss- und/oder Befeuchtungsvorgänge in beheizbaren Räumen insbesondere in Sauna und/oder und mindestens einem Schöpfelement zur Entnahme von Flüssigkeit aus dem Behälter, wobei mittels des Schöpfelements die aus dem Behälter entnommene Flüssigkeit einer Heiz- und/oder Verdampfungsvorrichtung zuführbar ist.

In Sauna und/oder Feucht-Warmluft-Kabinen ist es üblich, dass von den Benutzern oder dergleichen so genannte Ausgüsse durchgeführt werden. Hierbei handelt es sich um das sehr rasche Verdampfen einer auf die in einer Saunakabine befindlichen heißen Steine oder dergleichen mittels einer Schöpfkelle aufgegossenen Wassermenge. Diese Wassermenge wird mittels der Schöpfkelle aus einem in der Saunakabine neben dem Ofen aufgestellten Eimer entnommen.

Aufguss- und/oder Befeuchtungsvorrichtungen für Sauna- und/oder Feucht-Warmluft-Kabinen sind aus dem Stand der Technik bekannt. So sind beispielsweise Luftbefeuchtungsvorrichtungen wie z.B. in DE 20210006 offenbart bekannt, bei denen der Stiel den Schöpfkelle als Rinne ausgebildet ist und die Schöpfkelle im Bereich der Rinne mit Abstand von der Kelle schwenkbar um eine Achse gelagert ist, dergestalt, dass in einer Ruhestellung die Schöpfkelle in den Wasserbehälter eintaucht und in einer Aufgussstellung oberhalb des Wasserbehälters angeordnet ist, wodurch sich der Inhalt der Schöpfkelle über die Rinne über die heißen Steine entlehrt.

Aus der US 3,758,086 ist ein Luftbefeuchter bekannt, bei dem eine aus einem porösem Material gebildete Trommel durch einen Behälter mit Flüssigkeit geführt wird. Die Trommel ist auf einer Achse gelagert, wobei auf der Achse Schöpfelemente vorgesehen sind, die durch den Behälter hindurchgeführt werden und dem Behälter so entnommene Flüssigkeit einem Sammelbecken zur Ableitung bzw. Austausch der Flüssigkeit zuführen.

Nachteilig an den aus dem Stand der Technik bekannten Aufguss- bzw. Luftbefeuchtungsvorrichtungen ist jedoch, dass damit nur ein sehr unregelmäßiges Aufgussverhalten erreicht werden kann. Dies liegt insbesondere daran, dass die von der Schöpfkelle entnommene Wassermenge bzw. die im Flüssigkeitsbehälter angeordnete Flüssigkeitsmenge willkürlichen Schwankungen unterliegt und somit keine definierte Menge Flüssigkeit vom Schöpfmittel entnommen wird.

Aufgabe der vorliegenden Erfindung ist es, die oben beschriebenen Nachteile der aus dem Stand der Technik bekannten Aufguss- bzw. Luftbefeuchtungsvorrichtungen zu vermeiden und gleichzeitig eine Aufgussvorrichtung bzw. Befeuchtungsvorrichtung vorzuschlagen, welche ein gleichmäßiges Aufgussverhalten gestattet.

Diese Aufgabe wird durch eine Vorrichtung nach der Lehre des Anspruchs 1 gelöst.

Vorteilhafte Ausführungsformen der Erfindung sind Gegenstand der Unteransprüche.

Die Erfindung beruht auf dem Grundgedanken, ein gleichmäßiges Aufgussverhalten für Aufguss- und/oder Befeuchtungsvorgänge sicherzustellen, wozu das zur Vorrichtung gehörende Schöpfelement mittel- oder unmittelbar auf einer Achse diese umlaufend derart gelagert ist, dass es zumindest bereichsweise unter Aufnahme von Flüssigkeit durch einen Behälter hindurchgeführt werden kann. Dies hat den Vorteil, dass im Gegensatz zu anderen aus dem Stand der Technik bekannten Aufgussvorrichtungen, bei denen das Aufgießen mit einer Kelle aus einem Kübel heraus durch den Saunabadenden selbst oder durch einen Bade- oder Schwimmmeister durchgeführt wird, hierbei nahezu vollautomatisch erfolgen kann.

Das Schöpfelement selber, welches zur Entnahme von Flüssigkeit aus dem Behälter dient, kann dabei wannen-, schalen-, schaufel-, topf-, kellen- oder rinnenartig ausgebildet sein. Ein an der Vorrichtung vorgesehenes Verteilelement vermeidet ein unkontrolliertes Aufbringen der Flüssigkeit aus dem Schöpfelement direkt auf die Heiz- und/oder verdampfungsvorrichtung.

Eine Ausführungsform der Erfindung sieht vor, dass das Schöpfelement auf oder an einem Trägerelement angeordnet sein kann. Das Trägerelement selber kann dabei nach Art eines Rades und/oder einer Scheibe ausgebildet sein. Dies hat den Vorteil, dass eine definierte, um eine Achse verlaufende Schöpfposition des Schöpfelements erzielt werden kann.

Eine weitere Ausführungsform der Erfindung sieht vor, dass eine Mehrzahl von Schöpfelementen an oder auf dem Trägerelement angeordnet sind. Dadurch kann beispielsweise in einem Zusammenspiel mit einer zeitlich gesteuerten Drehgeschwindigkeit eine ständige Luftbefeuchtung erreicht werden, wodurch ein bevorzugtes Klima innerhalb der Sauna und/oder Feucht-Warmluft-Kabine erreicht wird.

Um eine konstante, selbständige Drehgeschwindigkeit des Trägerelements zu ermöglichen, kann vorgesehen sein, dass an der Vorrichtung eine elektrische Antriebsvorrichtung zum Antrieb des Schöpfelements und/oder des Trägerelements vorgesehen ist, wobei der Antrieb sowohl im Uhrzeigersinn als auch entgegengesetzt erfolgen kann.

Weiterhin ist an der Vorrichtung eine Zuführeinrichtung vorgesehen, mit der die vom Schöpfelement bzw. den Schöpfelementen aus dem Behälter entnommene Flüssigkeit dem der Heiz- und/oder Verdampfungsvorrichtung vorgelagerten Verteilelement zugeführt werden kann. Dies hat den Vorteil, dass der Heiz- und/oder Verdampfungsvorrichtung eine definierte Menge von Aufgussflüssigkeit zugeführt werden kann.

Um eine leichte Aufnahme bzw. kontrolliertes Weiterleiten der Flüssigkeit zu gewährleisten, kann es vorteilhaft sein, die Zuführeinrichtung rinnenartig auszubilden.

Das Verteilelement selber kann bevorzugt als Aufgussrinne oder Aufgussschale ausgebildet sein. Der Einsatz eines solchen Verteilelements hat den Vorteil, dass eine kontrollierte Abgabe der Flüssigkeit auf die Heiz- und/oder Verdampfungsvorrichtung gewährleistet werden kann. Des Weiteren kann durch ein solches Verteilelement gewährleistet werden, dass große Bereiche der Heiz- und/oder Verdampfungsvorrichtung von der Flüssigkeit befeuchtet werden, wodurch ein besseres Aufgussverhalten erreicht werden kann.

Um die Flüssigkeit aus dem Verteilelement an die Heiz- und/oder Verdampfungsvorrichtung weiterzuleiten, kann vorgesehen sein, dass das Verteilelement, insbesondere im Bereich seines Bodens, Ausnehmungen aufweist, durch die die zugeführte Flüssigkeit auf die im Wesentlichen unterhalb der Auffangmittel angeordnete Heiz- und/oder Verdampfungsvorrichtung abgegeben werden kann. Je nach gewünschter Abgabemenge der Flüssigkeit können diese Ausnehmungen größer oder kleiner gestaltet werden. Es ist somit möglich, an Hand von austauschbaren Verteilelementen verschiedene Aufgussverhalten zu erzielen.

Die Heiz- und/oder Verdampfungsvorrichtung selber kann im Wesentlichen aus einer Vielzahl von in einem Behältnis angeordneten erhitzbaren Steinen oder Ähnlichem bestehen.

Eine besonders bevorzugte Ausführungsform der Erfindung sieht vor, dass an der Vorrichtung eine Einrichtung vorgesehen sein kann, mit welcher der aus dem Behälter entnommenen Flüssigkeit vor Zuführung in das Verteilelement Zusatzstoffe zugeführt werden können. Mit Hilfe dieser Zusatzstoffe können verschiedene Effekte bzw. Aufgussverhalten realisiert werden.

Um die Zusatzstoffe dem Verteilelement zuzuführen, kann an der Vorrichtung eine Einrichtung vorgesehen sein, welche mindestens einen Vorratsbehälter und mindestens eine Dosiervorrichtung aufweist. Dadurch kann gewährleistet werden, dass ein nahezu vollautomatischer Zuführvorgang der Zusatzstoffe erreicht wird und darüber hinaus die abgegebene Menge an Zusatzstoffen einfach dosiert werden kann.

Bei den Zusatzstoffen selber kann es sich bevorzugt um Duftstoffe, ätherische Öle oder dergleichen handeln.

Um ein konstantes Flüssigkeitsniveau im Flüssigkeitsbehälter zu erreichen, kann vorgesehen sein, dass am oder im Flüssigkeitsbehälter eine insbesondere mechanisch oder elektrisch gesteuerte Niveauregulierung vorgesehen ist, mit dem der Flüssigkeitspegel des Flüssigkeitsbehälters regulierbar ist. Dies hat den Vorteil, dass eine konstante und genau definierte Wassermenge durch die Schöpfelemente aufgenommen werden kann. Hierdurch wird ein sehr gleichmäßiges Aufgussverhalten erreicht.

Um einen weitestgehend vollautomatischen Betrieb der Vorrichtung zu gewährleisten, kann vorgesehen sein, dass am Flüssigkeitsbehälter Anschlussmöglichkeiten für den Anschluss an ein Festwassernetz vorgesehen sind. Somit entfällt das aus dem Stand der Technik bekannte Auffüllen der Eimer bzw. Flüssigkeitsbehälter von Hand vollständig.

Eine weitere besonders bevorzugte Ausführungsform der Erfindung sieht vor, dass an der Vorrichtung Mittel zur Illumination vorgesehen sein können, wobei jeder Lichteffekt einem bestimmten Aufgussduft, Zusatzstoff und/oder Arbeitsgang zugeordnet sein kann. So kann die Vorrichtung neben ihrer Funktion als Aufguss- bzw. Befeuchtungsvorrichtung zusätzlich als Leuchtmittel, insbesondere zur Erzielung bestimmter Raumstimmungen, verwendet werden.

Eine weitere bevorzugte Ausführungsform der Erfindung sieht vor, dass an der Vorrichtung Mittel zur Erzielung akustischer Effekte vorgesehen sein können, wobei jeder akustische Effekt einem bestimmten Aufgussduft, Zusatzstoff und/oder Arbeitsgang zugeordnet ist.

Durch den Einsatz solcher Licht- bzw. akustischer Effekte kann ein nachvollziehbares und leicht zu kontrollierendes Aufgussverhalten der Vorrichtung sowie eine angenehme Raumstimmung gewährleistet werden.

Um einen weitestgehend vollautomatischen Betrieb der Vorrichtung zu gewährleisten, kann die Vorrichtung eine Steuereinheit aufweisen, mit der die Schöpfhäufigkeit, die Schöpfmenge, die Temperatur der Heiz- und/oder Verdampfungsvorrichtung, die Dosiereinheit für Zusatzstoffe, die Mittel zur Illumination und/oder die Mittel zur Erzielung akustischer Effekte programmablaufend oder ferngesteuert einstellbar bzw. an- oder abschaltbar sind.

Mittels einer Fernbedienung kann einer Person ferner der umständliche Gang zur Vorrichtung, beispielsweise für Aufgussvorgänge, erspart werden. Dadurch kann die Vorrichtung bedient werden, ohne dass eine Bedienperson die Saunakabine betreten muss.

Verschiedene Ausführungsformen der Erfindung sind in den Zeichnungen dargestellt und werden nachfolgend beispielhaft erläutert.

Es zeigen:
- **Fig.1**: eine Vorrichtung mit mehreren an einem Trägerelement an- geordneten Schöpfelementen;
- **Fig. 2**: eine Vorrichtung mit mehreren zwischen zwei Trägerele- menten angeordneten Schöpfmitteln;
- **Fig. 3**: eine Vorrichtung gemäß **Fig. 1** jedoch mit einer anders ge- stalteten Heiz- und/oder Verdampfungsvorrichtung;
- **Fig. 4**: eine Vorrichtung mit einem rinnenartigen Schöpfelement, angeordnet auf einem Trägerelement.

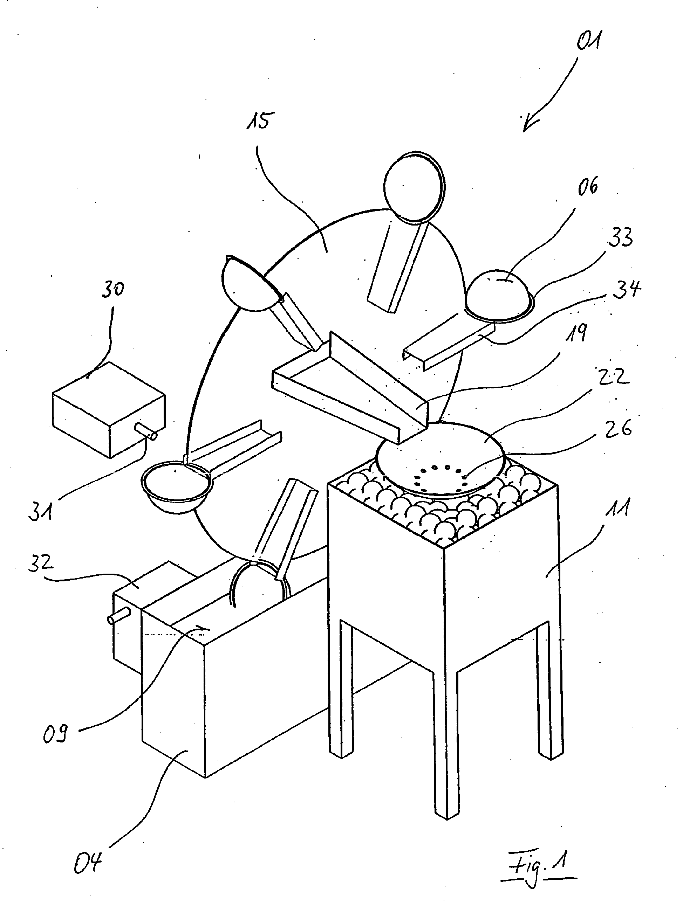

**Fig. 1** zeigt eine erfindungsgemäße Vorrichtung 01 in einer schematischen Darstellung. Die Vorrichtung 01 besteht aus mehreren Schöpfelementen 06, welche auf einem als Rad ausgebildeten Trägerelement 15 angeordnet sind. Das Trägerelement 15 ist auf einer nicht dargestellten Achse, die durch den Mittelpunkt des Trägerelements verläuft, diese umlaufend gelagert, wobei die Schöpfelemente 06, zumindest bereichsweise, unter Aufnahme yon Flüssigkeit 09 durch einen Behälter 04 hindurchgeführt werden können. Die Schöpfelemente 06 sind im Wesentlichen als Kombinationen aus Schalen (33)- und Rinnenelementen (34) ausgebildet. Die vom Schöpfelement 06 aufgenommene Flüssigkeit 09 kann über die am Schöpfelement 06 angeordnete Rinne (34) einer Zuführeinrichtung 19 zugeführt werden.

Über die Zuführeinrichtung 19 gelangt die Flüssigkeit 09 in ein unterhalb der Zuführeinrichtung 19 und oberhalb der Heiz- und/oder Verdampfungsvorrichtung gelagertes Verteilelement 22. Das Verteilelement 22 weist insbesondere im Bereich seines Bodens Ausnehmungen 26 auf, durch die die zugeführte Flüssigkeit 09 auf die unterhalb des Verteilelements 22 angeordnete Heiz- und/oder Verdampfungsvorrichtung 11, tropfend abgegeben werden kann.

Die Heiz- und/oder Verdampfungsvorrichtung 11 besteht im Wesentlichen aus einer Vielzahl von in einem Behältnis angeordneten, erhitzbaren Steinen.

Weiterhin ist an der Vorrichtung 01 eine Einrichtung 30 vorgesehen, mit welcher der aus dem Behälter 04 entnommenen Flüssigkeit 09, vor Zuführung in das Verteilelement 22, Zusatzstoffe zugeführt werden können. Dazu ist an der Einrichtung 30 eine Auslassöffnung 31 vorgesehen. Die Einrichtung 30 ist so gestaltet, dass sie mindestens einen nicht dargestellten Vorratsbehälter und mindestens eine nicht dargestellte Dosiervorrichtung aufweist.

Um einen gleichmäßigen Flüssigkeitspegel im Behälter 04 zu gewährleisten, ist am oder im Behälter 04 eine insbesondere mechanisch oder elektrisch gesteuerte Niveauregulierung 32 vorgesehen.

Ferner ist an der Vorrichtung 01 eine nicht dargestellte Steuereinheit vorgesehen, mit der die Schöpfhäufigkeit, die Schöpfmenge, die Temperatur der Heiz- und/oder Verdampfungsvorrichtung, die Dosiereinheit für Zusatzstoffe, sowie verschiedene Programm- und/oder Ablaufvorgänge ferngesteuert einstellbar bzw. an- oder abschaltbar sind.

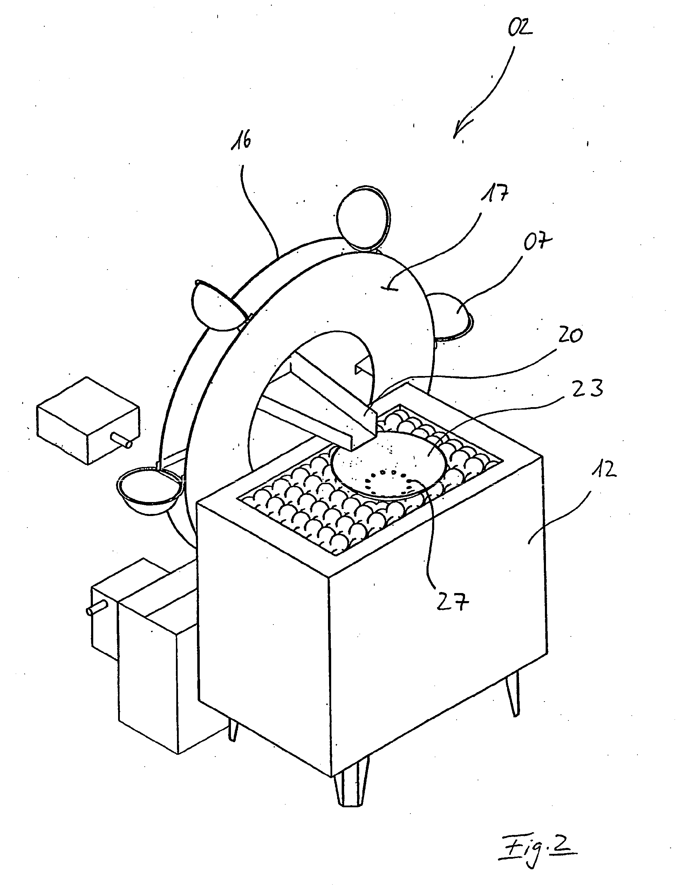

**Fig. 2** zeigt eine schematisch dargestellte Vorrichtung 02, wobei die Vorrichtung 02 hierbei eine Vielzahl von Schöpfelementen 07 aufweist, welche zwischen zwei Trägerelementen 16 und 17, welche in der Art eines Rades ausgebildet sind, angeordnet sind. Dabei ist das Trägerelement 17 so gestaltet, dass es eine Ausnehmung aufweist, durch welche die Zuführeinrichtung 20 ragt, welche so angeordnet ist, dass sie mit ihrer Auslassöffnung oberhalb des Verteilelements 23 mündet. Das Verteilelement 23 ist einer Heiz- und/oder Verdampfungsvorrichtung 12 vorgelagert, wobei im Verteilelement 23 eine Vielzahl von Ausnehmungen 27 vorgesehen sind.

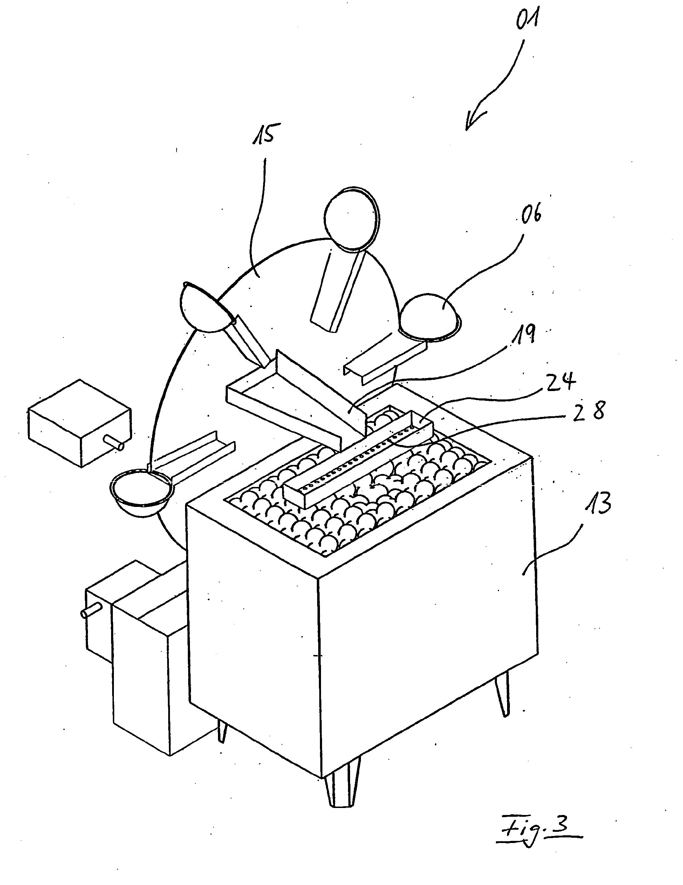

**Fig. 3** zeigt die Vorrichtung 01 gemäß **Fig. 1****,** wobei die Vorrichtung 01 hierbei mit einer anders gestalteten Heiz- und/oder Verdampfungsvorrichtung 13 zusammenwirkt. Bei dieser Ausführungsform ist das Verteilelement 24, welches unterhalb der Zuführeinrichtung 19 angeordnet ist, im Wesentlichen als Aufgussrinne ausgebildet. Die von den Schöpfelementen 06, welche auf dem Trägerelement 15 angeordnet sind, entnommene Flüssigkeit kann über die Zuführeinrichtung 19 in das als Aufgussrinne ausgebildete Verteilelement 24 abgegeben werden. Im Verteilelement 24 sind eine Vielzahl von Ausnehmungen 28 vorgesehen, durch welche die Flüssigkeit an die darunter vorgesehene Heiz- und/oder Verdampfungsvorrichtung 13 abgegeben werden kann.

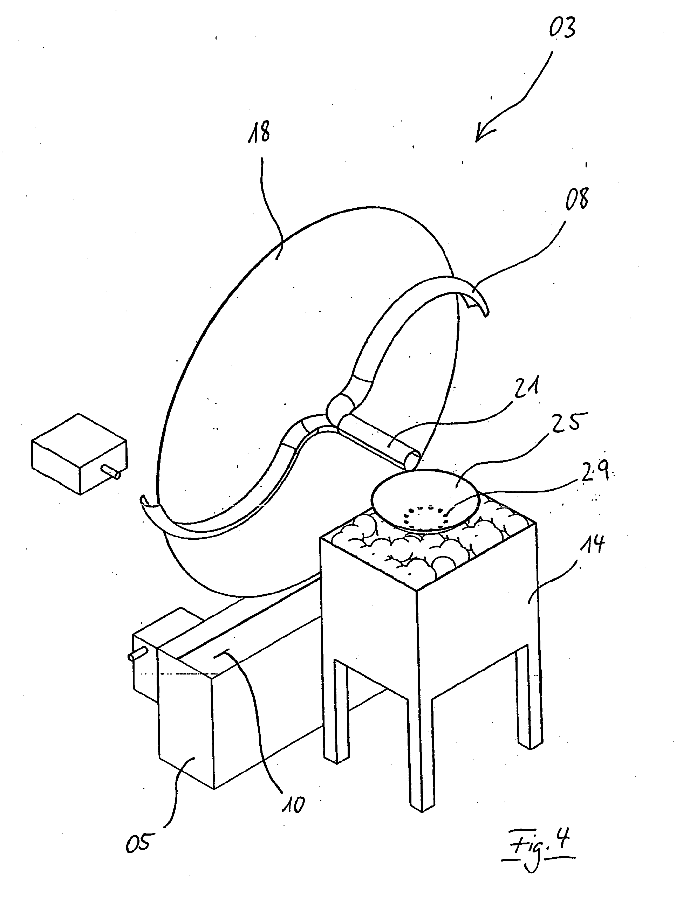

**Fig. 4** zeigt eine weitere Vorrichtung 03, wobei das Schöpfelement 08 hierbei im Wesentlichen rinnenartig ausgebildet ist. Das rinnenartige Schöpfelement 08 ist auf einem Trägerelement 18 angeordnet und auf diesem umlaufend drehbar auf einer nicht dargestellten Achse, die durch den Mittelpunkt des Trägerelements verläuft, gelagert. Die vom rinnenartigen Schöpfelement 08 entnommene Flüssigkeit 10, welche im Behälter 05 angeordnet ist, kann über eine mit dem rinnenartigen Schöpfelement 08 verbundene Zuführeinrichtung 21 an ein unterhalb der Zuführeinrichtung 21 vorgesehenes Verteilelement 25 abgegeben werden. Das Verteilelement 25 weist mehrere Ausnehmungen 29 auf, durch die die abgegebene Flüssigkeit 10 auf die unterhalb des Verteilelements angeordnete Heiz- und/oder Verdampfungsvorrichtung 14 abgegeben werden kann.

## Patentansprüche

1. Vorrichtung (01, 02, 03), geeignet für Aufguss- und/oder Befeuchtungsvorgänge in beheizbaren Räumen, insbesondere in Sauna- und/oder Feucht-Warmluft-Kabinen, mit einem mit Flüssigkeit (09, 10) gefüllten Behälter (04, 05) und mindestens einem Schöpfelement (06, 07, 08) zur Entnahme von Flüssigkeit (09, 10) aus dem Behälter (04, 05), wobei mittels des Schöpfelementes (06, 07, 08) die aus dem Behälter (04, 05) entnommene Flüssigkeit (09, 10) einer Heiz- und/oder Verdampfungsvorrichtung (11, 12, 13, 14) zuführbar ist,
**dadurch gekennzeichnet,**
**dass** das Schöpfelement (06, 07, 08) mittelbar oder unmittelbar auf einer Achse diese umlaufend derart gelagert ist, dass es zumindest bereichsweise unter Aufnahme von Flüssigkeit (09, 10) durch den Behälter (04, 05) hindurchgeführt werden kann, wobei die Vorrichtung (01, 02, 03) ein Verteilelement (22, 23, 24, 25) aufweist, welches vorgelagert der Heiz- und/oder Verdampfungsvorrichtung (11, 12, 13, 14) anordbar ist.

2. Vorrichtung nach Anspruch 1,
**dadurch gekennzeichnet,**
**dass** das Schöpfelement (06, 07, 08) wannen-, schalen-, schaufel-, topf-, kellen- oder rinnenartig ausgebildet ist.

3. Vorrichtung nach einem der Ansprüche 1 oder 2,
**dadurch gekennzeichnet,**
**dass** das Schöpfelement (06, 07, 08) auf oder an einem Trägerelement (15, 16, 17, 18) angeordnet ist.

4. Vorrichtung nach Anspruch 3,
**dadurch gekennzeichnet,**
**dass** das Trägerelement (15, 16, 17, 18) nach Art eines Rades und/oder einer Scheibe ausgebildet ist.

5. Vorrichtung nach einem der Ansprüche 1 bis 4,
**dadurch gekennzeichnet,**
**dass** eine Mehrzahl von Schöpfelementen (06, 07, 08) an oder auf dem Trägerelement (15, 16, 17, 18) angeordnet ist.

6. Vorrichtung nach einem der Ansprüche 1 bis 5,
**gekennzeichnet durch**
eine insbesondere elektrische Antriebsvorrichtung zum Antrieb des Schöpfelementes (06, 07, 08) und/oder des Trägerelements (15, 16, 17, 18), wobei der Antrieb sowohl im Uhrzeigersinn als auch entgegengesetzt erfolgen kann.

7. Vorrichtung nach einem der Ansprüche 1 bis 6,
**gekennzeichnet durch**
eine Zuführeinrichtung (19, 20, 21), mit der die vom Schöpfelement (06, 07, 08) bzw. den Schöpfelementen (06, 07, 08) aus dem Behälter (04, 05) entnommene Flüssigkeit (09, 10) dem der Heiz- und/oder Verdampfungsvorrichtung (11, 12, 13 ,14) vorgelagerten Verteilelement (22, 23, 24, 25) zugeführt wird.

8. Vorrichtung nach Anspruch 7,
**dadurch gekennzeichnet,**
**dass** die Zuführeinrichtung (19, 20, 21) rinnenartig ausgebildet ist.

9. Vorrichtung nach einem der Ansprüche 7 oder 8,
**dadurch gekennzeichnet,**
**dass** das Verteilelement (22, 23, 24, 25) als Aufgussrinne oder Aufgussschale ausgebildet ist.

10. Vorrichtung nach einem der Ansprüche 7 bis 9,
**dadurch gekennzeichnet,**
**dass** das Verteilelement (22, 23, 24, 25) insbesondere im Bereich seines Bodens Ausnehmungen (26, 27, 28) aufweist, durch die die zugeführte Flüssigkeit (09, 10), auf die im Wesentlichen unterhalb der Verteilelemente (22, 23, 24, 25) angeordnete Heiz- und/oder Verdampfungsvorrichtung (11, 12, 13, 14), abgegeben werden kann.

11. Vorrichtung nach einem der Ansprüche 1 bis 10,
**dadurch gekennzeichnet,**
**dass** die Heiz- und/oder Verdampfungsvorrichtung (11, 12, 13, 14) im Wesentlichen aus einer Vielzahl von in einem Behältnis angeordneten erhitzbaren Steinen oder Ähnlichem besteht.

12. Vorrichtung nach einem der Ansprüche 1 bis 11,
**dadurch gekennzeichnet,**
**dass** an der Vorrichtung (01, 02, 03) eine Einrichtung (30) vorgesehen ist, mit welcher der aus dem Behälter (04, 05) entnommenen Flüssigkeit (09, 10) vor Zuführung in das Verteilelement (22, 23, 24, 25) Zusatzstoffe zugeführt werden können.

13. Vorrichtung nach Anspruch 12,
**dadurch gekennzeichnet,**
**dass** die Einrichtung (30) mindestens einen Vorratsbehälter und mindestens eine Dosiervorrichtung aufweist.

14. Vorrichtung nach einem der Ansprüche 12 oder 13,
**dadurch gekennzeichnet,**
**dass** es sich bei den Zusatzstoffen um Duftstoffe, ätherische Öle oder dergleichen handelt.

15. Vorrichtung nach einem der Ansprüche 1 bis 14,
**dadurch gekennzeichnet,**
**dass** am oder im Behälter (04, 05) eine insbesondere mechanisch oder elektrisch gesteuerte Niveauregulierung (32) vorgesehen ist, mit dem der Flüssigkeitspegel des Behälters regulierbar ist.

16. Vorrichtung nach einem der Ansprüche 1 bis 15,
**dadurch gekennzeichnet,**
**dass** am Behälter (04, 05) Anschlussmöglichkeiten für den Anschluss an ein Festwassernetz vorgesehen sind.

17. Vorrichtung nach einem der Ansprüche 1 bis 16,
**dadurch gekennzeichnet,**
**dass** an der Vorrichtung (01, 02, 03) Mittel zur Illumination vorgesehen sind, wobei jeder Lichteffekt einem bestimmten Aufgussduft, Zusatzstoff und/oder Arbeitsgang zugeordnet sein kann.

18. Vorrichtung nach einem der Ansprüche 1 bis 27,
**dadurch gekennzeichnet,**
**dass** an der Vorrichtung (01, 02, 03) Mittel zur Erzielung akustischer Effekte vorgesehen sind, wobei jeder akustische Effekt einem bestimmten Aufgussduft, Zusatzstoff und/oder Arbeitsgang zugeordnet sein kann.

19. Vorrichtung nach einem der Ansprüche 1 bis 21,
**dadurch gekennzeichnet,**
**dass** die Vorrichtung (01, 02, 03) eine Steuereinheit aufweist, mit der die Schöpfhäufigkeit, die Schöpfmenge, die Temperatur der Heiz- und/oder Verdampfungsvorrichtung, die Dosiereinheit für Zusatzstoffe, die Mittel zur Illumination und/oder die Mittel zur Erzielung akustischer Effekte programm-, ablauf- oder ferngesteuert einstellbar bzw. an- oder abschaltbar sind.

## Claims

1. A device (01, 02, 03), suitable for wetting and/or humidifying processes in heatable spaces, in particular in sauna and/or steam rooms, comprising a container (04, 05), which is filled with a liquid (09, 10) and at least one ladle element (06, 07, 08) for removing liquid (09, 10) from the container (04, 05), wherein the liquid (09, 10), which is removed from the container (04, 05) by means of the ladle element (06, 07, 08), can be supplied to a heating and/or vaporization device (11, 12, 13, 14), **characterized in**
**that** the ladle element (06, 07, 08) is supported directly or indirectly on an axis so as to rotate about it such that it can be guided through the container (04, 05) at least area by area while picking up liquid (09, 10), wherein the device (01, 02, 03) encompasses a distributor element (22, 23, 24, 25), which can be arranged upstream of the heating and/or vaporization device (11, 12, 13, 14).

2. The device according to claim 1,
**characterized in**
**that** the ladle element (06, 07, 08) is embodied in a tub-like, bowl-like, shovel-like, pot-like, scoop-like or channel-like manner.

3. The device according to one of claims 1 or 2,
**characterized in**
**that** the ladle element (06, 07, 08) is arranged on or at a support element (15, 16, 17, 18).

4. The device according to claim 3,
**characterized in**
**that** the support element (15, 16, 17, 18) is embodied according to the type of a wheel and/or of a disk.

5. The device according to one of claims 1 to 4,
**characterized in**
**that** a plurality of ladle elements (06, 07, 08) is arranged at or on the support element (15, 16, 17, 18).

6. The device according to one of claims 1 to 5,
**characterized by**
an in particular electric drive device for driving the ladle element (06, 07, 08) and/or the support element (15, 16, 17, 18), wherein the drive can be carried out clockwise as well as counter-clockwise.

7. The device according to one of claims 1 to 6,
**characterized by**
a supply device (19, 20, 21), by mean of which the liquid (09, 10) removed from the container (04, 05) by means of the ladle element (06, 07, 08) or the ladle elements (06, 07, 08), respectively, is supplied to the distributor element (22, 23, 24, 25), which is arranged upstream of the heating and/or vaporization device (11, 12, 13, 14).

8. The device according to claim 7,
**characterized in**
**that** the supply device (19, 20, 21) is embodied in a channel-like manner.

9. The device according to one of claims 7 or 8,
**characterized in**
**that** the distributor element (22, 23, 24, 25) is embodied as wetting channel or wetting bowl.

10. The device according to one of claims 7 to 9,
**characterized in**
**that** the distributor element (22, 23, 24, 25), in particular in the area of its base, encompasses recesses (26, 27, 28), through which the supplied liquid (09, 10) can be passed to the heating and/or vaporization device (11, 12, 13, 14), which is arranged substantially below the distributor elements (22, 23, 24, 25).

11. The device according to one of claims 1 to 10,
**characterized in**
**that** the heating and/or vaporization device (11, 12, 13, 14) substantially consist of a plurality of heatable stones or the like, which are arranged in a container.

12. The device according to one of claims 1 to 11,
**characterized in**
**that** provision is made at the device (01, 02, 03) for an arrangement (30), by means of which additives can be supplied to the liquid (09, 10), which is removed from the container (04, 05), prior to the supply into the distributor element (22, 23, 24, 25).

13. The device according to claim 12,
**characterized in**
**that** the arrangement (30) encompasses at least one storage container and at least one metering device.

14. The device according to one of claims 12 or 13,
**characterized in**
**that** the additives are fragrances, essential oils or the like.

15. The device according to one of claims 1 to 14,
**characterized in**
**that** provision is made at or in the container (04, 05) for an in particular mechanically or electrically controlled level regulation (32), by means of which the liquid level of the container can be regulated.

16. The device according to one of claims 1 to 15,
**characterized in**
**that** provision is made at the container (04, 05) for connection options for connection to a fixed water network.

17. The device according to one of claims 1 to 16,
**characterized in**
**that** provision is made at the device (01, 02, 03) for means for illumination, wherein each light effect can be assigned to a certain wetting fragrance, additive and/or operation.

18. The device according to one of claims 1 to 17,
**characterized in**
**that** provision is made at the device (01, 02, 03) for means for attaining acoustic effects, wherein each acoustic effect can be assigned to a certain wetting fragrance, additive and/or operation.

19. The device according to one of claims 1 to 18,
**characterized in**
**that** the device (01, 02, 03) encompasses a control unit, by means of which the ladling frequency, the ladling quantity, the temperature of the heating and/or vaporization device, the metering unit for additives, the means for illumination and/or the means for attaining acoustic effects can be adjusted or turned on or off, respectively, in a program-controlled, process-controlled or remote-controlled manner.

## Revendications

1. Dispositif (01, 02, 03) adapté pour des processus d'infusion et/ou d'humidification dans des locaux susceptibles d'être chauffés, notamment dans des cabines de sauna et/ou d'air chaud/humide, avec un récipient (04, 05) rempli de liquide (09, 10) et au moins un élément de puisage (06, 07, 08) pour prélever du liquide (09, 10) dans le récipient (04, 05), au moyen de l'élément de puisage (06, 07, 08) le liquide (09, 10) prélevé dans le récipient (04, 05) étant susceptible d'être amené dans un dispositif de chauffage et/ou d'évaporation (11, 12, 13, 14), **caractérisé en ce que**
l'élément de puisage (06, 07,08) est logé indirectement ou directement sur un axe en tournant autour de ce dernier, de telle sorte qu'on puisse au moins le faire passer par endroits à travers le récipient (04, 05) en prélevant du liquide (09, 10), le dispositif (01, 02, 03) comportant un élément distributeur (22, 23, 24, 25) lequel est susceptible d'être disposé en étant placé en amont du dispositif de chauffage et/ou d'évaporation (11, 12, 13, 14).

2. Dispositif selon la revendication 1,
**caractérisé en ce que**
l'élément de puisage (06, 07, 08) est conçu sous la forme d'un bac, d'une coupe, d'une pelle, d'un pot, d'une louche ou d'une goulotte.

3. Dispositif selon l'une quelconque des revendications 1 ou 2,
**caractérisé en ce que**
l'élément de puisage (06, 07, 08) est disposé sur le dessus de ou sur élément support (15, 16, 17, 18).

4. Dispositif selon la revendication 3,
**caractérisé en ce que**
l'élément support (15, 16, 17, 18) est conçu à la manière d'une roue et/ou d'un disque.

5. Dispositif selon l'une quelconque des revendications 1 à 4,
**caractérisé en ce qu'**
une pluralité d'éléments de puisage (06, 07, 08) est disposée sur le ou sur le dessus de l'élément support (15, 16, 17, 18).

6. Dispositif selon l'une quelconque des revendications 1 à 5,
**caractérisé par**
un dispositif d'entraînement notamment électrique pour entraîner l'élément de puisage (06, 07, 08) et/ou l'élément support (15, 16, 17, 18), l'entraînement pouvant s'effectuer aussi bien dans le sens horaire, qu'à l'opposée.

7. Dispositif selon l'une quelconque des revendications 1 à 6,
**caractérisé par**
un dispositif d'alimentation (19, 20, 21), à l'aide duquel le liquide (09, 10) prélevé dans le récipient (04, 05) par l'élément de puisage (06, 07, 08) est alimenté vers l'élément de distribution (22, 23, 24, 25) placé en amont du dispositif de chauffage et/ou d'évaporation (11, 12, 13, 14).

8. Dispositif selon la revendication 7,
**caractérisé en ce que**
le dispositif d'alimentation (19, 20, 21) est conçu sous la forme d'une goulotte.

9. Dispositif selon l'une quelconque des revendications 7 ou 8,
**caractérisé en ce que**
l'élément de distribution (22, 23, 24, 25) est conçu sous la forme d'une goulotte d'infusion ou d'une coupe d'infusion.

10. Dispositif selon l'une quelconque des revendications 7 à 9,
**caractérisé en ce que**
l'élément de distribution (22, 23, 24, 25) comporte notamment dans la zone de son fond des évidements (26, 27, 28), à travers lesquels le liquide (09, 10) alimenté peut être restitué sur le dispositif de chauffage et/ou d'évaporation (11, 12, 13, 14) sensiblement disposé sous les éléments de distribution (22, 23, 24, 25).

11. Dispositif selon l'une quelconque des revendications 1 à 10,
**caractérisé en ce que**
le dispositif de chauffage et/ou d'évaporation (11, 12, 13, 14) consiste sensiblement dans une pluralité de pierres chauffantes ou analogues disposées dans un contenant.

12. Dispositif selon l'une quelconque des revendications 1 à 11,
**caractérisé en ce que**
sur le dispositif (01, 02, 03) est prévu un système (30) permettant d'ajouter des adjuvants au liquide (09, 10) prélevé dans le récipient (04, 05), avant de l'alimenter dans l'élément de distribution (22, 23, 24, 25).

13. Dispositif selon la revendication 12,
**caractérisé en ce que**
le système (30) comporte au moins un récipient de réserve et au moins un dispositif de dosage.

14. Dispositif selon l'une quelconque des revendications 12 ou 13,
**caractérisé en ce que**
les adjuvants sont des substances odoriférantes, des huiles essentielles ou similaires.

15. Dispositif selon l'une quelconque des revendications 1 à 14,
**caractérisé en ce que**
sur ou dans le récipient (04, 05), il est prévu une régulation de niveau (32) à commande notamment mécanique ou électrique, permettant d'ajuster le niveau de liquide dans le récipient.

16. Dispositif selon l'une quelconque des revendications 1 à 15,
**caractérisé en ce que**
sur le récipient (04, 05) sont prévues des possibilités de raccordement, pour le raccorder sur un réseau d'eau courante.

17. Dispositif selon l'une quelconque des revendications 1 à 16,
**caractérisé en ce que**
sur le dispositif (01, 02, 03) sont prévus des moyens d'illumination, chaque effet lumineux pouvant être associé à un certain parfum d'infusion, à un certain adjuvant et/ou à une certaine étape de travail.

18. Dispositif selon l'une quelconque des revendications 1 à 17,
**caractérisé en ce que**
sur le dispositif (01, 02, 03) sont prévus des moyens de production d'effets acoustiques, chaque effet acoustique pouvant être associé à un certain parfum d'infusion, à un certain adjuvant et/ou à une certaine étape de travail.

19. Dispositif selon l'une quelconque des revendications 1 à 18,
**caractérisé en ce que**
le dispositif (01, 02, 03) comporte une unité de commande, permettant de régler par programmation, par commande séquentielle ou par télécommande ou de mettre en marche ou à l'arrêt la fréquence de puisage, la quantité puisée, la température du dispositif de chauffage et/ou d'évaporation, l'unité de dosage des adjuvants, les moyens d'illumination et/ou les moyens de productions d'effets acoustiques.
